# EUROPEAN PATENT SPECIFICATION

(11) **EP 0 584 279 B1**
(45) Date of publication and mention of the grant of the patent: **14.03.2001**
(21) Application number: 92913964.0
(22) Date of filing: 14.05.1992
(51) Int. Cl.: A61K 47/48

(54) **TARGETED DELIVERY OF GENES ENCODING IMMUNOGENIC PROTEINS**
GERICHTETE ABGABE VON GENEN, DIE IMMUNOGENE PROTEINE KODIEREN
APPORT CIBLE DE GENES CODANT DES PROTEINES IMMUNOGENES

(30) Priority: 14.05.1991 US 699891
(43) Date of publication of application: 02.03.1994
(73) Proprietor: THE UNIVERSITY OF CONNECTICUT, Storrs, CT 06269 (US); THE IMMUNE RESPONSE CORPORATION, Carlsbad, CA 92008 (US)
(72) Inventor: WU, George, Y., Bloomfield, CT 06002 (US); SPITALNY, George, L., Cheshire, CT 06410 (US); FINDEIS, Mark, A., Meriden, CT 06450 (US)
(74) Representative: UEXKÜLL & STOLBERG
(86) International application number: US9203875
(87) International publication number: WO9220316

(56) References cited:
- EP-A- 0 161 188
- WO-A-92/05250
- JOURNAL OF BIOLOGICAL CHEMISTRY, vol. 264, no. 29, 15 October 1989, BALTIMORE, US, pages 16985-16987; WU ET AL.: 'TARGETING GENES:DELIVERY AND PERSISTENT EXPRESSION OF A FOREIGN GENE DRIVEN BY MAMMALIAN REGULATORY ELEMENTS IN VIVO'
- JOURNAL OF BIOLOGICAL CHEMISTRY, vol. 266, no. 6, 25 February 1991, BALTIMORE, US, pages 3361-3364; KATO ET AL.: 'EXPRESSION OF HEPATITIS B VIRUS SURFACE ANTIGEN IN ADULT RAT LIVER'

## Description

### Background of the Invention

Immunization of animals for the purpose of vaccination or production of antibodies (used for passive immunization, diagnostic or scientific reagents) typically involves injection of a natural or recombinant protein which has been partially or completely purified to homogeneity. Purification of the protein to homogeneity usually requires several steps involving anionic, cationic and molecular sieve chromatography. These procedures are time consuming and the homogeneity of the final product must be verified. Alternatively, a differentiating assay which can distinguish the protein of choice from all other proteins can be used to develop a monoclonal antibody.

Methods to target a soluble molecular complex comprising a gene to hepatocytes for expression of the gene within these cells have been disclosed, for example in Wu et al., J. Biol. Chem. (1989), 254: 16985-16987, and in WO 92/05250. The present invention however discloses the targeting to cells of a gene encoding an immunogenic protein or polypeptide, specifically. Accordingly the invention can be used to vaccinate mammals and to produce antibodies for experimental therapeutic or diagnostic use.

W090/11092 teaches the delivery of a naked (i.e. unconjugated) gene to a cell by directly injecting the gene in a pharmaceutically acceptable carrier, for example, into the interstitial space of a tissue comprising the cell, so that the gene is taken up into the interior of the cell. According to the present invention, however, a gene encoding an immunogenic protein or polypeptide is targeted to a specific cell in the form of a soluble molecular complex.

### Summary of the Invention

This invention pertains to a soluble molecular complex for targeting, a gene (or genes) encoding an immunogenic protein or polypeptide for which an immune response is desired, to a specific cell in a host organism in vivo and obtaining expression of the gene, production of the gene-encoded protein or polypeptide and development of an immune response against the immunogenic protein or polypeptide in the host organism. The molecular complex comprises a nucleic acid molecule containing an expressible gene encoding a desired immunogenic protein or polypeptide complexed with a carrier which is a conjugate of a cell-specific binding agent which is not an antibody and a gene-binding agent. The cell-specific binding agent is specific for a cellular surface structure, typically a receptor, which mediates internalization of bound ligands by endocytosis, such as the asialoglycoprotein receptor of hepatocytes. The cell-specific binding agent can be a natural or synthetic ligand (for example, a protein, polypeptide, glycoprotein, etc.) which specifically binds a cellular surface structure which then mediates internalization of the bound complex. The gene-binding component of the conjugate is a compound such as a polycation which stably complexes the gene under extracellular conditions and releases the gene under intracellular conditions so that it can function within the cell.

The complex of the gene and the carrier is stable and soluble in physiological fluids. It can be administered in vivo where it is selectively taken up by the target cell via the surface-structure-mediated endocytotic pathway. The incorporated gene is expressed, the gene-encoded protein or polypeptide is processed and secreted as a soluble or a cell surface protein or polypeptide by the transfected cell and an immune response is evoked against the protein or polypeptide in the host organism.

The soluble molecular complex of this invention can be used to elicit an immune response in an organism to a desired immunogenic protein or polypeptide. It can be used for immunization of organisms for the purpose of vaccination or for the production of antibodies for experimental (e.g., research reagent), diagnostic or therapeutic use.

### Brief Description of the Figure

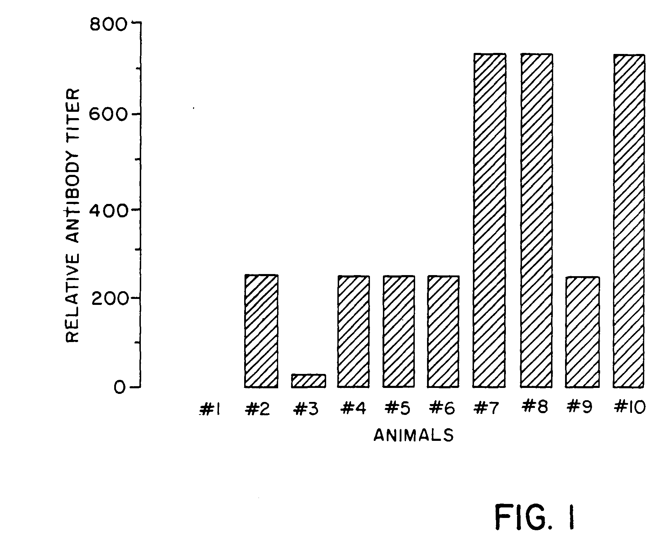

Figure 1 is a bar graph representing the relative titer of anti-HBsAg antibody in animals receiving the molecular complex of the invention.

### Detailed Description of the Invention

A soluble, targetable molecular complex is used to deliver a gene encoding an immunogenic protein or polypeptide to a target cell or tissue in vivo and obtain expression of the gene, production of the gene-encoded protein or polypeptide and development of an immune response against the immunogenic protein or polypeptide in a host organism. The molecular complex comprises the gene encoding a desired immunogenic protein or polypeptide to be delivered complexed with a carrier made up of a binding agent specific for the target cell and a gene-binding agent.

The gene, generally in the form of DNA, encodes the desired immunogenic protein or polypeptide. Typically, the gene comprises a structural gene encoding the immunogenic protein or polypeptide in a form suitable for processing and secretion as a soluble or cell surface protein or polypeptide by the target cell. For example, the gene encodes appropriate signal sequences which direct processing and secretion of the protein or polypeptide. The signal sequence may be the natural sequence of the protein or exogenous sequences. The structural gene is linked to appropriate genetic regulatory elements required for expression of the gene-encoded protein or polypeptide by the target cell. These include a promoter and optionally an enhancer element operable in the target cell. The gene can be contained in an expression vector such as a plasmid or a transposable genetic element along with the genetic regulatory elements necessary for expression of the gene and secretion of the gene-encoded product.

The carrier component of the complex is a conjugate of a cell-specific binding agent and a gene-binding agent which is not an antibody. The cell-specific binding agent specifically binds a cellular surface structure which mediates internalization by, for example, the process of endocytosis. The surface structure can be a protein, polypeptide, carbohydrate, lipid or combination thereof. It is typically a surface receptor which mediates endocytosis of a ligand. Thus, the binding agent can be a natural or synthetic ligand which binds the receptor. The ligand can be a protein, polypeptide, glycoprotein or glycopeptide which has functional groups that are exposed sufficiently to be recognized by the cell surface structure. It can also be a component of a biological organism such as a virus, cells (e.g., mammalian, bacterial, protozoan) or artificial carriers such as liposomes.

Ligands useful in forming the carrier will vary according to the particular cell to be targeted. For targeting hepatocytes, glycoproteins having exposed terminal carbohydrate groups such as asialoglycoprotein (galactose-terminal) can be used, although other ligands such as polypeptide hormones may also be employed. Examples of asialoglycoproteins include asialoorosomucoid, asialofetuin and desialylated vesicular stomatitis virus. Such ligands can be formed by chemical or enzymatic desialylation of glycoproteins that possess terminal sialic acid and penultimate galactose residues. Alternatively, asialoglycoprotein ligands can be formed by coupling galactose terminal carbohydrates such as lactose or arabinogalactan to non-galactose bearing proteins by reductive lactosamination.

For targeting the molecular complex to other cell surface receptors, other types of ligands can be used, such as mannose for macrophages, mannose-6-phosphate glycoproteins for fibroblasts, intrinsic factor-vitamin B12 for enterocytes and insulin for fat cells. Alternatively, the cell-specific binding agent can be a receptor or receptor-like molecule, which binds a ligand (e.g., internalizing antigen) on the cell surface.

The gene-binding agent complexes the gene to be delivered. Complexation with the gene must be sufficiently stable in vivo to prevent significant uncoupling of the gene extracellularly prior to internalization by the target cell. However, the complex is cleavable under appropriate conditions within the cell so that the gene is released in functional form. For example, the complex can be labile in the acidic and enzyme rich environment of lysosomes. A noncovalent bond based on electrostatic attraction between the gene-binding agent and the gene provides extracellular stability and is releasable under intracellular conditions.

Preferred gene-binding agents are polycations that bind negatively charged polynucleotides. These positively charged materials can bind noncovalently with the gene to form a soluble, targetable molecular complex which is stable extracellularly but releasable intracellularly. Suitable polycations are polylysine, polyarginine, polyornithine, basic proteins such as histones, avidin, protamines and the like. A preferred polycation is polylysine. Other noncovalent bonds that can be used to releasably link the expressible gene include hydrogen bonding, hydrophobic bonding, electrostatic bonding alone or in combination such as, anti-polynucleotide antibodies bound to polynucleotide, and strepavidin or avidin binding to polynucleotide containing biotinylated nucleotides.

The carrier can be formed by chemically linking the cell-specific binding agent and the gene-binding agent. The linkage is typically covalent. A preferred linkage is a peptide bond. This can be formed with a water soluble carbodiimide as described by Jung, G. et al. (1981) Biochem. Biophys. Res. Commun. 101:599-606. An alternative linkage is a disulfide bond.

The linkage reaction can be optimized for the particular cell-specific binding agent and gene-binding agent used to form the carrier. Reaction conditions can be designed to maximize linkage formation but to minimize the formation of aggregates of the carrier components. The optimal ratio of cell-specific binding agent to gene-binding agent can be determined empirically. When polycations are used, the molar ratio of the components will vary with the size of the polycation and the size of the cell-specific binding agent. When a protein such as asialoorosomucoid and a polycation such as polylysine are used, the mass ratio of protein to polycation will typically be in the range of 5:1 to 1:5, preferably around 1:1. The molar ratios in these mixtures will vary widely with the molecular weights of the components. For the same protein, a greater number of molar equivalents of a lower molecular weight polycation will be required to prepare a conjugate with the same cation content as a conjugate prepared with a higher molecular weight polycation. Uncoupled components and aggregates can be separated from the carrier by molecular sieve chromatography or ion-exchange chromatography or a combination of the two techniques.

The gene encoding the desired immunogenic protein or polypeptide can be complexed to the carrier by combining these two components in solution containing NaCl at a concentration of 0.15 to 2 M. The carrier is added to the DNA and if a higher NaCl concentration is used, the solution of complex is dialyzed to reduce the salt concentration. In a preferred method, the carrier and DNA are both in 0.15 M NaCl and the carrier is added to the DNA to form a complex directly.

The gene encoding the desired immunogenic protein or polypeptide can be complexed to the carrier by a stepwise dialysis procedure. In a preferred method, for use with carriers made of polycations such as polylysine, the dialysis procedure begins with a 2 M NaCl dialyzate and ends with a 0.15 M NaCl solution. See e.g., Wu, G.Y. and Wu, C.H. J. Biol. Chem. (1987) 262:4429-4432. The gradually decreasing NaCl concentration results in binding of the DNA to the carrier.

The molecular complex can contain more than one copy of the same gene or one or more different genes. Preferably, the mass ratio of carrier to polynucleotide is about 1:5 to 5:1, preferably about 2:1. This ratio will vary considerably depending upon the components of the complex. For example, a conjugate with a lower proportion of polycation will typically be used at a higher ratio to the DNA.

The molecular complex of this invention can be administered parenterally. Preferably, it is injected intravenously. The complex is administered in solution in a physiologically acceptable vehicle.

Cells can be transfected in vivo for transient expression and secretion of the gene-encoded product. For prolonged expression and secretion, the gene can be administered repeatedly. Alternatively, the transfected target cell can be stimulated to replicate by surgical or pharmacological means to prolong expression of the incorporated gene. See, for example, U.S. Patent Application Serial No. 588,013, filed September 25, 1990 (corresponding to WO-A-9205250).

The molecular complex of this invention is adaptable for delivery of a wide range of genes to a specific cell or tissue. Preferably, the complex is targeted to the liver by exploiting the hepatic asialoglycoprotein receptor system which allows for in vivo transfection of hepatocytes by the process of receptor-mediated endocytosis. The liver has the highest rate of protein synthesis per gram of tissue. Thus, the molecular complex of this invention can be used to specifically target the liver as a site for high efficiency production of an immunogenic protein or polypeptide to thereby elicit an immune response to the protein or polypeptide.

The immunogen is typically, but not necessarily, a protein or polypeptide foreign to the host. It can be any protein or polypeptide which contains an epitope or epitopes (B or T cell), which are immunogenic in a host organism. For vaccination, the immunogen can be an immunogenic component or components of a pathogen, such as a virus, bacterium, or parasite, which can elicit a protective immune response against the pathogen. For example, the immunogen can be an envelope protein of a virus (e.g., HIV glycoprotein, HBV surface antigen) or a cell wall constituent of a bacterium.

In some cases, the protein or polypeptide against which the immune response is desired may be nonimmunogenic or poorly immunogenic. Such proteins or polypeptides can be coupled to an immunogenic carrier protein. This can be accomplished genetically by preparing a chimeric gene that encodes a fusion of the protein or polypeptide and the carrier.

The immunogen can be any agent against which antibodies are desired for diagnostic or therapeutic purposes. For example, the immunogen can be a component of a pathogen which elicits antibodies and/or cell useful for passive immunization against the pathogen. The immunogen can be a cell surface structure associated with a diseased cell such as tumor-associated antigen which elicits anti-tumor antibodies for diagnosis or therapy of tumor. The immunogen can also be any agent against which antibodies are needed for diagnostic assays. Examples include pathogens, hormones, cytokines, metabolites or drugs.

The method of this invention can be used to vaccinate an organism (human or other animal) to provide protection against infection. In one embodiment, a gene encoding an immunogenic protein or polypeptide which is a component of a pathogen is complexed to a conjugate of an asialoglycoprotein and a polycation. The resulting soluble complex is administered to a host organism to target liver cells in amounts sufficient to selectively transfect the cells and to provide sufficient production and secretion of the immunogen to elicit a protective immune response against the immunogen in the organism.

The method of this invention can also be used to produce polyclonal or monoclonal antibodies. For production of a polyclonal antibody, a gene encoding the immunogenic protein or polypeptide complexed with a carrier is administered to an organism (e.g., mouse, rabbit or goat) to elicit an immune response (antibody and/or cellular) against the immunogenic protein or polypeptide. Antiserum which is specific for the immunogen is obtained from the immunized organism by known techniques.

For production of monoclonal antibodies, spleen cells or other antibody-producing cells are obtained from an organism immunized with the molecular complex. These cells are fused with appropriate immortalizing cells, such as myelomas, to produce hybridomas. The hybridomas are screened and those which produce antibodies specific for the immunogenic protein or polypeptide are selected.

In vivo gene transfer of an immunogenic protein or polypeptide to immunize an organism has several advantages over injection of a natural or recombinant protein which has been partially or completely purified to homogenity. Expression and secretion of an immunogenic protein or polypeptide in vivo eliminates the necessity of purifying an immunogen and can invoke a polyclonal antibody response that will be monospecific for the immunogenic protein or polypeptide. In addition, continuous expression and secretion of the immunogenic protein or polypeptide can boost the initial immune response in an organism.

This invention is illustrated by the following exemplification.

### Exemplification

### General

Polylysines (PL), obtained as the hydrobromide salts, and 3'dimethylaminopropyl ethyl carbodiimide (EDC) were from Sigma Chemical Co. Acrodisc syringe filters were obtained from Gelman Sciences. Orosomucoid was isolated from pooled human plasma as described by Whitehead and Sammons (Biochim. Biophys. Acta (1966) 124:209-211) and desialylated to form asialoorosomucoid (ASOR) by treatment with 0.1 N H₂SO₄ at 80°C for 1 hour (Schmid et al. (1967) Biochem. J. 104:361-368). ASOR concentrations were determined using an OD₂₈₀ of 0.92 for a 1.0 mg/mL solution in water. DNA concentrations were determined using an OD₂₈₀ of 1.0 for a 50 µg/mL solution in water. HPLC purification of ASOR-PL conjugates was performed using a Brownlee Aquapore CX-300 cation exchange column (10 mm x 25 cm) obtained from Rainin Instrument Co. Dialysis tubing was obtained from Spectrum Medical Industries.

### Synthesis of asialoorosomucoid-polylysine (ASOR-PL) conjugate

ASOR (60 mg/8 mL), PolyLysine HBr (5200 MW, 92 mg/3 mL) and EDC (42 mg/2 mL) were each dissolved in water. Each solution was filtered (0.45 µm) and the filter was washed with 2, 1 and 1 mL of water, respectively, which was combined with the corresponding solution. The pH's of the ASOR and the Polylysine solutions were adjusted to 7.4 with 132 µL and 420 µL of 0.1 N NaOH respectively. The EDC solution plus a 1 mL rinse was added to the ASOR solution with stirring and the pH of the mixture adjusted to 7.4 with 4 µL of 0.1 N NaOH. The polylysine solution plus a 1 mL rinse with water was added with stirring to the ASOR-EDC solution and the pH adjusted to 7.4 with 92 µL of 0.1 N NaOH. The reaction mixture was incubated for 24 hours at 37°C. The reaction mixture was then placed in 12,000-14,000 MW cut-off dialysis tubing and dialyzed against 20L of water for 24 hours and then twice against 8L of water for 3 hours. The resulting solution was lyophilized to yield 58 mg of ASOR-PL conjugate (38.0% based on ASOR and polylysine combined masses). Based on the OD₂₈₀ of a 1.0 mg/mL solution in water (0.68) this conjugate was determined to be 74% ASOR by weight.

### Alternative procedures

ASOR-PL conjugates prepared with polylysines of different molecular weights (Mr = 21,000 and 69,000) have also produced DNA complexes that produce similar positive results in animal experiments. These conjugates are synthesized in essentially the same manner as the conjugates made with 5.2Kd polylysine, with the additional step after dialysis of purification by HPLC followed by dialysis and lyophilization. The HPLC purification is performed on a Brownlee Aquapore cation exchange column (10 mm x 25 cm) with a flow rate of 4.5 mL/min. The column was eluted with buffers containing 0.1 M sodium acetate adjusted to acidic pH with HC1. A gradient from pH 5.0 to 2.0 was used to elute unconjugated ASOR, conjugate and polylysine. Conjugates typically elute at pH 2.5 to 2.0. In some runs, conjugate and excess polylysine overlap. In those instances, the front portion of the main peak that is rich in ASOR, as determined by the higher ratio of the absorbances at 280 nm and 230 nm, is collected and used for DNA complex formation as described above.

### Gel retardation assays

A plasmid containing a head-to-tail dimer of the complete hepatitis B viral genome citation was made up to 100 µg/mL in 0.3 M NaCl and filtered (0.2 µm). An aliquot of the DNA solution (5 µL, 500 ng DNA) was placed in each of sixteen 1.5 mL polypropylene microtubes. A 1 mg/mL stock of ASOR-PL (5.2Kd polylysine) conjugate in water was prepared and filtered (0.2 µm). Solutions of the conjugate diluted with water were added to the DNA samples to create conjugate to DNA ratios in the range from 0.2:1 to 3.0:1 (w/w). Water was added to each sample to bring the final DNA concentration to 50 µg/mL and the NaCl concentration to 0.15 M. The samples were vortexed briefly, centrifuged for 30 seconds at 14,000 rpm and incubated at room temperature for 1 hour. Loading buffer (3.3 µL of 40% sucrose, 0.25% bromophenol blue) was added to each sample which was then loaded on to a 1% agarose gel. The gel was prepared by dissolving 0.4 g of agarose in 40 mL of TPE buffer (90 mM TRIS-phosphate, 2 mM EDTA, pH 8.0), adding 0.5 µL of a 10 mg/mL solution of ethidium bromide. The gel was 8 x 6.5 x 0.7 cm, the running buffer used was TPE buffer and a constant 50 V was applied for 1.5 hours. Extent of retardation of DNA was then observed under UV light. Full retardation was taken as that ratio of conjugate to DNA that retained all of the DNA in the sample well of the gel.

### DNA-Conjugate complex formation

To plasmid (870 µL of a 0.2 µm-filtered solution at 915 µg/mL) was added water (523 µL) and 4.0 M NaCl to give a final DNA concentration of 500 µg/mL and a final NaCl concentration of 0.15-0.5 M. This sample was placed in a 5 mL vial and stirred. To ASOR-PL (5.2Kd polylysine) conjugate (597 µL of a 0.2 µm filtered solution at 2.0 mg/mL) was added water (796 µL) and 4.0 M NaCl (199 µL) to give a final conjugate concentration of 750 µg/mL and a final NaCl concentration of 0.15-0.5 M. The conjugate was added to the DNA solution at 6.7 µL/min (0.4 mL/h) via a peristaltic pump. After complete addition the plasmid DNA/ASOR-PL complex was filtered (0.2 µm) and determined by UV to contain 250 µg/mL of DNA. Complexation was verified by running an agarose gel of plasmid DNA (500 ng) and the complex (500 ng of DNA).

### In vivo transfection of animals and detection of HBsAg

BALB/c mice 8-16 weeks of age, purchased from Harlan Sprague Dawley, Inc. (Madison, WI), were injected intravenously with 34 µg to 125 µg of HBV DNA contained in a DNA/ASOR-PL complex. Fifteen - 30 minutes after injection, mice were anesthetized with ether and a 2/3 partial hepatectomy performed to induce a more prolonged state of gene expression. For this procedure a midline incision was made from just below the sternum extending midway down the abdomen. From the opening in the peritoneal cavity the liver was exposed and surgical silk was wrapped around the major bottom lobe of the liver. The silk was tied off just below the point where the hepatic veins join the inferior vena cava. The ligated region of the liver was excised and the incision closed with wound clips.

Serum samples were collected from mice at varying intervals after injection of the DNA/ASOR-PL complex and initially tested for the presence of hepatitis B surface antigen in the circulation using an ELISA kit from Abbott Laboratories (Abbott Park, IL). This test can detect as little as 5 pico grams of antigen.

### Enzyme-linked immunosorbant assay for detection of antibodies against HBsAg

Each well of an Immulon-4 96 well plate (Fisher Scientific) was coated with 500 ng/well of HBsAg (provided by Catherine Wu, University of Connecticut). For binding, HBsAg was suspended in 0.01 M sodium bicarbonate buffer, pH 9.6 and incubated for 60 minutes at 37°C. The wells were washed 5X with phosphate buffered saline containing 0.025% Tween 20 (PBS-Tween). Blocking of unreacted sites was accomplished by adding to each well 200 pl of 2.5% bovine serum albumin w/v (Sigma) suspended in PBS; incubation was carried out at 37°C for 1-2 hours. The wells were again washed 5X with PBS-Tween. Sera from each of the animals was initially diluted 1:3 in PBS and 100 µl added to each well. To each of the wells in the vertical row the serum was diluted 3 fold in successive wells. Serum samples and dilutions were incubated for 1 hour at 37°C. Each well was again washed 5X with PBS-Tween. To each well 100 µl of a 1:1000 dilution of goat antibody against mouse IgG heavy and light chain, labeled with horse radish peroxidase (HRP), was added and incubated for 1 hour at 37°C. The wells were again washed 5X with PBS-Tween. Finally, to each well 100 µl of tetramethylbenzidene (TMB) peroxidase substrate (Kirkegard and Perry, Gaithersburg, MD) was added and blue color was allowed to develop for 15-30 minutes. The development of the catalyzed color reaction was evaluated spectrophotometrically with a plate reader.

### Results

Pre-injection serum samples tested negative for HBsAg. Following intravenous injection of the DNA/ASOR-PL complex, sera from animals were tested for the presence of HBsAg. In 8 out of 10 animals antigen was detected in the sera of animals as early as 3 days or as late as 7 days after injection. All animals which tested positive for the presence of HBsAg became negative for HBsAg 7-10 days after first detection antigen. Sera from these animals were then tested for the presence of antibodies against HBsAg. The antibody titers are presented in Figure 1 as the reciprocal dilution of the sera which was above background levels detected by the plate reader. The results in Figure 1 show that 8 out of 10 animals developed antibodies against HBsAg ranging in relative antibody titer from about 250 to 700. Animal #3 showed an antibody titer of about 50 and in animal #1 no HBsAg or antibody was detected. This data shows that an immune response can be evoked by method of this invention.

It should be noted that animal #3 tested on two separate occasions (days 4 and 10 after injection of DNA/ASOR-PL) remained negative for HBsAg, but developed antibodies against HBsAg. The ELISA test for HBsAg has a sensitivity of about 5 pg. Typically, larger amounts of exogenously administered immunogens are required to evoke an immune response in an organism. This suggests that the introduction of a foreign protein via endogenous production in host cells may be a more efficient process for evoking an immune response than by injecting foreign protein directly into animals.

## Claims (Claims for the following Contracting State(s): AT, BE, CH, DE, DK, FR, GB, IT, LI, LU, MC, NL, SE)

1. A soluble molecular complex for targeting a gene encoding an immunogenic protein or polypeptide to a specific cell, the complex comprising nucleic acid containing an expressible gene encoding the immunogenic protein or polypeptide in a form suitable for secretion as a soluble or cell surface protein or polypeptide by the target cell complexed with a carrier comprising a cell-specific binding agent, which is not an antibody, and a gene-binding agent.

2. A soluble molecular complex of claim 1, wherein the nucleic acid is a DNA.

3. A soluble molecular complex of claims 1 or 2, wherein the immunogenic protein or polypeptide is a component of a pathogen.

4. A soluble molecular complex of claims 1 to 3, wherein the gene-binding agent is a polycation.

5. A soluble molecular complex of claim 4, wherein the polycation is polylysine.

6. A soluble molecular complex of claims 1 to 5. wherein the cell-specific binding agent binds a surface receptor of the cell which mediates endocytosis.

7. A soluble molecular complex of claim 6, wherein the cell-specific binding agent is a ligand for an asialoglycoprotein receptor.

8. A soluble molecular complex of claim 7, wherein the ligand is an asialoglycoprotein and the targeted cell is a hepatocyte.

9. A soluble molecular complex of claims 1 to 8, wherein the expressible gene is complexed with the gene-binding agent by a noncovalent bond.

10. A soluble molecular complex of claims 1 to 9, wherein the cell-specific binding agent is linked to the gene-binding agent by a covalent bond.

11. A soluble molecular complex of claims 1 to 10, wherein the expressible gene is complexed with the gene-binding agent so that the gene is released in functional form under intracellular conditions.

12. A therapeutic composition comprising a solution of the molecular complex of claims 1 to 11 and a physiologically acceptable vehicle.

13. A soluble molecular complex of claims 1 to 12, wherein the immunogenic protein or polypeptide is hepatitis B surface antigen.

14. A soluble molecular complex of claims 1 to 13, wherein the gene is contained in an expression vector along with genetic regulatory elements necessary for expression of the gene by a hepatocyte.

15. A soluble molecular complex of claim 14, wherein the expression vector is a plasmid or viral DNA.

16. Use of a soluble molecular complex according to claims 1 to 15 for the manufacture of a medicament for use in immunization against the immunogenic protein or polypeptide.

## Claims (Claims for the following Contracting State(s): ES, GR)

1. A method of producing a soluble molecular complex for targeting a gene encoding an immunogenic protein or polypeptide to a specific cell, wherein the nucleic acid containing an expressible gene encoding the immunogenic protein or polypeptide in a form suitable for secretion as a soluble or cell surface protein or polypeptide by the target cell is complexed with a carrier comprising a cell-specific binding agent, which is not an antibody, and a gene-binding agent.

2. A method of producing a soluble molecular complex according to claim 1, wherein the nucleic acid is a DNA.

3. A method of producing a soluble molecular complex according to claims 1 or 2, wherein the immunogenic protein or polypeptide is a component of a pathogen.

4. A method of producing a soluble molecular complex according to claims 1 to 3, wherein the gene-binding agent is a polycation.

5. A method of producing a soluble molecular complex according to claim 4, wherein the polycation is polylysine.

6. A method of producing a soluble molecular complex according to claims 1 to 5. wherein the cell-specific binding agent binds a surface receptor of the cell which mediates endocytosis.

7. A metohd of producing a soluble molecular complex according to claim 6, wherein the cell-specific binding agent is a ligand for an asialoglycoprotein receptor.

8. A method of producing a soluble molecular complex according to claim 7, wherein the ligand is an asialoglycoprotein and the targeted cell is a hepatocyte.

9. A method of producing a soluble molecular complex according to claims 1 to 8, wherein the expressible gene is complexed with the gene-binding agent by a noncovalent bond.

10. A method of producing a soluble molecular complex according to claims 1 to 9, wherein the cell-specific binding agent is linked to the gene-binding agent by a covalent bond.

11. A method of producing a soluble molecular complex according to claims 1 to 10, wherein the expressible gene is complexed with the gene-binding agent so that the gene is released in functional form under intracellular conditions.

12. A method of producing atherapeutic composition, wherein a solution of the molecular complex prepared according to claims 1 to 11 is mixed with a physiologically acceptable vehicle.

13. A method of producing a soluble molecular complex according to claims 1 to 12, wherein the immunogenic protein or polypeptide is hepatitis B surface antigen.

14. A method of producing a soluble molecular complex according to claims 1 to 13, wherein the gene is contained in an expression vector along with genetic regulatory elements necessary for expression of the gene by a hepatocyte.

15. A method of producing a soluble molecular complex according to claim 14, wherein the expression vector is a plasmid or viral DNA.

16. Use of a soluble molecular complex prepared according to claims 1 to 15 for the manufacture of a medicament for use in immunization against the immunogenic protein or polypeptide.

## Patentansprüche (Patentansprüche für folgende(n) Vertragsstaat(en): AT, BE, CH, DE, DK, FR, GB, IT, LI, LU, MC, NL, SE)

1. Löslicher Molekülkomplex zum Dirigieren (targeting) eines für ein immunogenes Protein oder Polypeptid kodierenden Gens zu einer spezifischen Zelle, wobei der Komplex eine Nukleinsäure umfaßt, die ein exprimierbares Gen enthält, das für das immunogene Protein oder Polypeptid in einer Form kodiert, die für Sekretion als ein lösliches oder Zelloberflächenprotein oder -polypeptid durch die Zielzelle geeignet ist, komplexiert mit einem Trägerstoff, der ein zellspezifisches Bindungsmittel, das kein Antikörper ist, und ein Gen-bindendes Mittel umfaßt.

2. Löslicher Molekülkomplex nach Anspruch 1, bei dem die Nukleinsäure eine DNA ist.

3. Löslicher Molekülkomplex nach den Ansprüchen 1 oder 2, bei dem das immunogene Protein oder Polypeptid ein Bestandteil eines Pathogens ist.

4. Löslicher Molekülkomplex nach den Ansprüchen 1 bis 3, bei dem das Gen-bindende Mittel ein Polykation ist.

5. Löslicher Molekülkomplex nach Anspruch 4, bei dem das Polykation Polylysin ist.

6. Löslicher Molekülkomplex nach den Ansprüchen 1 bis 5, bei dem das zellspezifische Bindungsmittel einen Oberflächenrezeptor auf der Zelle bindet, der Endozytose vermittelt.

7. Löslicher Molekülkomplex nach Anspruch 6, bei dem das zellspezifische Bindungsmittel ein Ligand für einen Asialoglykoproteinrezeptor ist.

8. Löslicher Molekülkomplex nach Anspruch 7, bei dem der Ligand ein Asialoglykoprotein ist und die Zielzelle ein Hepatozyt ist.

9. Löslicher Molekülkomplex nach den Ansprüchen 1 bis 8, bei dem das exprimierbare Gen mit dem Gen-bindenden Mittel durch eine nicht-kovalente Bindung komplexiert ist.

10. Löslicher Molekülkomplex nach den Ansprüchen 1 bis 9, bei dem das zellspezifische Bindungsmittel mit dem Gen-bindenden Mittel durch eine kovalente Bindung verbunden ist.

11. Löslicher Molekülkomplex nach den Ansprüchen 1 bis 10, bei dem das exprimierbare Gen mit dem Gen-bindenden Mittel derart komplexiert ist, daß das Gen in funktioneller Form unter intrazellulären Bedingungen abgegeben wird.

12. Therapeutische Zusammensetzung umfassend eine Lösung des Molekülkomplexes nach den Ansprüchen 1 bis 11 und eine physiologisch akzeptables Vehikel.

13. Löslicher Molekülkomplex nach den Ansprüchen 1 bis 12, bei dem das immunogene Protein oder Polypeptid Hepatitis-B-Oberflächen-Antigen ist.

14. Löslicher Molekülkomplex nach den Ansprüchen 1 bis 13, bei dem das Gen in einem Expressionsvektor zusammen mit genetischen Regulationselementen enthalten ist, die für die Expression des Gens durch einen Hepatozyten notwendig sind.

15. Löslicher Molekülkomplex nach Anspruch 14, bei dem der Expressionsvektor ein Plasmid oder virale DNA ist.

16. Verwendung eines löslichen Molekülkomplexes nach den Ansprüchen 1 bis 15 für die Herstellung eines Medikamentes zur Verwendung bei der Immunisierung gegen das immunogene Protein oder Polypeptid.

## Patentansprüche (Patentansprüche für folgende(n) Vertragsstaat(en): ES, GR)

1. Verfahren zur Herstellung eines löslichen Molekülkomplexes zum Dirigieren (targeting) eines für ein immunogenes Protein oder Polypeptid kodierenden Gens zu einer spezifischen Zelle, wobei der Komplex eine Nukleinsäure umfaßt, die ein exprimierbares Gen enthält, das für das immunogene Protein oder -polypeptid in einer Form kodiert, die für Sekretion als ein lösliches oder Zelloberflächenprotein oder Polypeptid durch die Zielzelle geeignet ist, mit einem Trägerstoff komplexiert wird, der ein zellspezifisches Bindungsmittel, das kein Antikörper ist, und ein Gen-bindendes Mittel umfaßt.

2. Verfahren zur Herstellung eines löslichen Molekülkomplexes nach Anspruch 1, bei dem die Nukleinsäure eine DNA ist.

3. Verfahren zur Herstellung eines löslichen Molekülkomplexes nach den Ansprüchen 1 oder 2, bei dem das immunogene Protein oder Polypeptid ein Bestandteil eines Pathogens ist.

4. Verfahren zur Herstellung eines löslichen Molekülkomplexes nach den Ansprüchen 1 bis 3, bei dem das Gen-bindende Mittel ein Polykation ist.

5. Verfahren zur Herstellung eines löslichen Molekülkomplexes nach Anspruch 4, bei dem das Polykation Polylysin ist.

6. Verfahren zur Herstellung eines löslichen Molekülkomplexes nach den Ansprüchen 1 bis 5, bei dem das zellspezifische Bindungsmittel einen Oberflächenrezeptor auf der Zelle bindet, der Endozytose vermittelt.

7. Verfahren zur Herstellung eines löslichen Molekülkomplexes nach Anspruch 6, bei dem das zellspezifische Bindungsmittel ein Ligand für einen Asialoglykoproteinrezeptor ist.

8. Verfahren zur Herstellung eines löslichen Molekülkomplexes nach Anspruch 7, bei dem der Ligand ein Asialoglykoprotein ist und die Zielzelle ein Hepatozyt ist.

9. Verfahren zur Herstellung eines löslichen Molekülkomplexes nach den Ansprüchen 1 bis 8, bei dem das exprimierbare Gen mit dem Gen-bindenden Mittel durch eine nicht-kovalente Bindung komplexiert ist.

10. Verfahren zur Herstellung eines löslichen Molekülkomplexes nach den Ansprüchen 1 bis 9, bei dem das zellspezifische Bindungsmittel mit dem Gen-bindenden Mittel durch eine kovalente Bindung verbunden ist.

11. Verfahren zur Herstellung eines löslichen Molekülkomplexes nach den Ansprüchen 1 bis 10, bei dem das exprimierbare Gen mit dem Gen-bindenden Mittel derart komplexiert ist, daß das Gen in funktioneller Form unter intrazellulären Bedingungen abgegeben wird.

12. Verfahren zur Herstellung einer therapeutischen Zusammensetzung, bei dem eine Lösung eines gemäß den Ansprüchen 1 bis 11 hergestellten Molekülkomplexes, mit einem physiologisch akzeptablen Vehikel gemischt wird.

13. Verfahren zur Herstellung eines löslichen Molekülkomplexes nach den Ansprüchen 1 bis 12, bei dem das immunogene Protein oder Polypeptid Hepatitis-B-Oberflächen-Antigen ist.

14. Verfahren zur Herstellung eines löslichen Molekülkomplexes nach den Ansprüchen 1 bis 13, bei dem das Gen in einem Expressionsvektor zusammen mit genetischen Regulationselementen enthalten ist, die für die Expression des Gens durch einen Hepatozyten notwendig sind.

15. Verfahren zur Herstellung eines löslichen Molekülkomplexes nach Anspruch 14, bei dem der Expressionsvektor ein Plasmid oder virale DNA ist.

16. Verwendung eines gemäß den Ansprüchen 1 bis 15 hergestellten löslichen Molekülkomplexes, für die Herstellung eines Medikamentes zur Verwendung bei der Immunisierung gegen das immunogene Protein oder Polypeptid.

## Revendications (Revendications pour l'(les) Etat(s) contractant(s) suivant(s): AT, BE, CH, DE, DK, FR, GB, IT, LI, LU, MC, NL, SE)

1. Complexe moléculaire soluble destiné à diriger un gène codant pour une protéine ou un polypeptide immunogène vers une cellule spécifique, le complexe comprenant un acide nucléique contenant un gène susceptible d'être exprimé codant pour la protéine ou le polypeptide immunogène sous une forme appropriée à sa sécrétion sous la forme d'une protéine ou d'un polypeptide soluble ou de surface cellulaire par la cellule cible, complexé avec un support comprenant un agent de liaison spécifique de la cellule, qui n'est pas un anticorps, et un agent de liaison au gène.

2. Complexe moléculaire soluble selon la revendication 1, l'acide nucléique étant de l'ADN.

3. Complexe moléculaire soluble selon la revendication 1 ou 2, la protéine ou le polypeptide immunogène étant un composant d'un agent pathogène.

4. Complexe moléculaire soluble selon les revendications 1 à 3, l'agent de liaison au gène étant un polycation.

5. Complexe moléculaire soluble selon la revendication 4, le polycation étant la polylysine.

6. Complexe moléculaire soluble selon les revendications 1 à 5, l'agent de liaison spécifique de la cellule se liant à un récepteur de surface de la cellule qui médie l'endocytose.

7. Complexe moléculaire soluble selon la revendication 6, l'agent de liaison spécifique de la cellule étant un ligand pour un récepteur d'une asialoglycoprotéine.

8. Complexe moléculaire soluble selon la revendication 7, le ligand étant une asialoglycoprotéine et la cellule cible un hépatocyte.

9. Complexe moléculaire soluble selon les revendications 1 à 8, le gène susceptible d'être exprimé étant complexé avec l'agent de liaison au gène par une liaison non covalente.

10. Complexe moléculaire soluble selon les revendications 1 à 9, l'agent de liaison spécifique de la cellule étant lié à l'agent de liaison au gène par une liaison non covalente.

11. Complexe moléculaire soluble selon les revendications 1 à 10, le gène susceptible d'être exprimé étant complexé avec l'agent de liaison au gène de manière à ce que le gène soit libéré sous une forme fonctionnelle dans les conditions intracellulaires.

12. Composition thérapeutique comprenant une solution du complexe moléculaire soluble selon les revendications 1 à 11 et un véhicule acceptable sur le plan physiologique.

13. Complexe moléculaire soluble selon les revendications 1 à 12, la protéine ou le polypeptide immunogène étant l'antigène de surface de l'hépatite B.

14. Complexe moléculaire soluble selon les revendications 1 à 13, le gène étant contenu dans un vecteur d'expression et accompagné des éléments de régulation génétiques nécessaires à l'expression du gène par un hépatocyte.

15. Complexe moléculaire soluble selon la revendication 14, le vecteur d'expression étant un plasmide ou de l'ADN viral.

16. Utilisation d'un complexe moléculaire soluble selon les revendications 1 à 15 dans la fabrication d'un médicament destiné à être utilisé pour procéder à une immunisation contre la protéine ou le polypeptide immunogène.

## Revendications (Revendications pour l'(les) Etat(s) contractant(s) suivant(s): ES, GR)

1. Procédé de production d'un complexe moléculaire soluble destiné à diriger un gène codant pour une protéine ou un polypeptide immunogène vers une cellule spécifique, dans lequel l'acide nucléique contenant un gène susceptible d'être exprimé codant pour la protéine ou le polypeptide immunogène sous une forme appropriée à sa sécrétion sous la forme d'une protéine ou d'un polypeptide soluble ou de surface cellulaire par la cellule cible est complexé avec un support comprenant un agent de liaison spécifique de la cellule, qui n'est pas un anticorps, et un agent de liaison au gène.

2. Procédé de production d'un complexe moléculaire soluble selon la revendication 1, l'acide nucléique étant de l'ADN.

3. Procédé de production d'un complexe moléculaire soluble selon la revendication 1 ou 2, la protéine ou le polypeptide immunogène étant un composant d'un agent pathogène.

4. Procédé de production d'un complexe moléculaire soluble selon les revendications 1 à 3, l'agent de liaison au gène étant un polycation.

5. Procédé de production d'un complexe moléculaire soluble selon la revendication 4, le polycation étant la polylysine.

6. Procédé de production d'un complexe moléculaire soluble selon les revendications 1 à 5, l'agent de liaison spécifique de la cellule se liant à un récepteur de surface de la cellule qui médie l'endocytose.

7. Procédé de production d'un complexe moléculaire soluble selon la revendication 6, l'agent de liaison spécifique de la cellule étant un ligand pour un récepteur d'une asialoglycoprotéine.

8. Procédé de production d'un complexe moléculaire soluble selon la revendication 7, le ligand étant une asialoglycoprotéine et la cellule cible un hépatocyte.

9. Procédé de production d'un complexe moléculaire soluble selon les revendications 1 à 8, le gène susceptible d'être exprimé étant complexé avec l'agent de liaison au gène par une liaison non covalente.

10. Procédé de production d'un complexe moléculaire soluble selon les revendications 1 à 9, l'agent de liaison spécifique de la cellule étant lié à l'agent de liaison au gène par une liaison non covalente.

11. Procédé de production d'un complexe moléculaire soluble selon les revendications 1 à 10, le gène susceptible d'être exprimé étant complexé avec l'agent de liaison au gène de manière à ce que le gène soit libéré sous une forme fonctionnelle dans les conditions intracellulaires.

12. Procédé de production d'une composition thérapeutique comprenant une solution du complexe moléculaire soluble selon les revendications 1 à 11 et un véhicule acceptable sur le plan physiologique.

13. Procédé de production d'un complexe moléculaire soluble selon les revendications 1 à 12, la protéine ou le polypeptide immunogène étant l'antigène de surface de l'hépatite B.

14. Procédé de production d'un complexe moléculaire soluble selon les revendications 1 à 13, le gène étant contenu dans un vecteur d'expression et accompagné des éléments de régulation génétiques nécessaires à l'expression du gène par un hépatocyte.

15. Procédé de production d'un complexe moléculaire soluble selon la revendication 14, le vecteur d'expression étant un plasmide ou de l'ADN viral.

16. Utilisation d'un complexe moléculaire soluble préparé selon les revendications 1 à 15 dans la fabrication d'un médicament destiné à être utilisé pour procéder à une immunisation contre la protéine ou le polypeptide immunogène.
